(19) Europäisches Patentamt
European Patent Office
Office européen des brevets

(11) Veröffentlichungsnummer: **0 084 774**
**A1**

(12) **EUROPÄISCHE PATENTANMELDUNG**

(21) Anmeldenummer: **83100044.3**

(22) Anmeldetag: **05.01.83**

(51) Int. Cl.³: **C 07 D 253/06**, A 01 N 43/64 // C07C121/34

(30) Priorität: **15.01.82 DE 3201110**

(43) Veröffentlichungstag der Anmeldung: **03.08.83**
**Patentblatt 83/31**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Kranz, Eckart, Dr., Am Acker 9, D-5600 Wuppertal 1 (DE)**
Erfinder: **Findeisen, Kurt, Dr., In der Follmühle 10, D-5068 Odenthal 2 (DE)**
Erfinder: **Eue, Ludwig, Dr., Paul-Klee-Strasse 36, D-5090 Leverkusen 1 (DE)**
Erfinder: **Schmidt, Robert, Dr., Im Waldwinkel 110, D-5060 Bergisch-Gladbach 2 (DE)**

(54) **3-Alken(in)yl-mercapto(amino)-4-amino-6-tert.-butyl-1,2,4-triazin-5-one, Verfahren zu ihrer Herstellung sowie ihrer Verwendung als Herbizide.**

(57) Neue 3-Alkenylmercapto-, 3-Alkinylmercapto-, 3-Alkenylamino- und 3-Alkinylamino-4-amino-6-tert.-butyl-1,2,4-triazin-5-one der allgemeinen Formel I

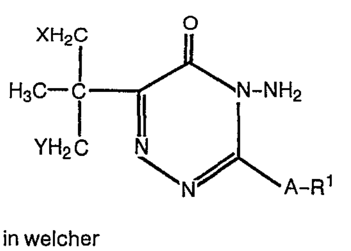

(I)

in welcher
A für Schwefel oder den Rest $-NR^2-$ steht,
$R^2$ für Wasserstoff oder Alkyl steht,
$R^1$ für Alkenyl oder Alkinyl steht,
X für Halogen oder Alkoxy steht und
Y für Wasserstoff, Halogen oder Alkoxy steht,
Verfahren zu ihrer Herstellung und ihre Verwendung als Herbizide.



ACTORUM AG

0084774

BAYER AKTIENGESELLSCHAFT 5090 Leverkusen, Bayerwerk
Zentralbereich Bi/Hed
Patente, Marken und Lizenzen Ib

# 3-Alken(in)yl-mercapto(amino)-4-amino-6-tert.-butyl-1,2,4-triazin-5-one, Verfahren zu ihrer Herstellung sowie ihrer Verwendung als Herbizide.

Die vorliegende Erfindung betrifft neue 3-Alkenylmercapto-, 3-Alkinylmercapto-, 3-Alkenylamino- und 3-Alkinylamino-4-amino-6-tert.-butyl-1,2,4-triazin-5-one, Verfahren zu ihrer Herstellung sowie ihre Verwendung als Herbizide, insbesondere als selektive Herbizide.

Es ist bereits bekannt geworden, daß substituierte 1,2,4-Triazin-5-one, wie zum Beispiel das 4-Amino-3-methylmercapto-6-tert.-butyl-1,2,4-triazin-5-on, als Herbizide verwendet werden können (vgl. z.B. DE-PS 1795 784 und US-PS 3 671 523). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

0084774

Es wurden neue 3-Alken(in)yl-mercapto(amino)-4-amino-6-tert.-butyl-1,2,4-triazin-5-one der allgemeinen Formel I

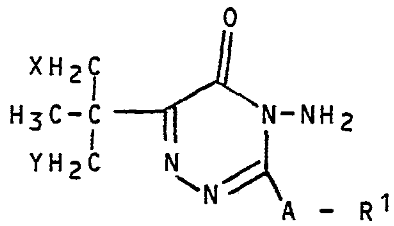

(I)

in welcher

A für Schwefel oder den Rest $-NR^2-$ steht,

$R^2$ für Wasserstoff oder Alkyl steht,

$R^1$ für Alkenyl oder Alkinyl steht,

X für Halogen oder Alkoxy steht und

Y für Wasserstoff, Halogen oder Alkoxy steht,

gefunden.

Weiterhin wurde gefunden, daß man die 3-Alken(in)yl-mercapto(amino)-4-amino-6-tert.-butyl-1,2,4-triazin-5-one der Formel (I) erhält, wenn man

a) 4-Amino-3-mercapto-6-tert.-butyl-1,2,4-triazin-5-one der Formel II

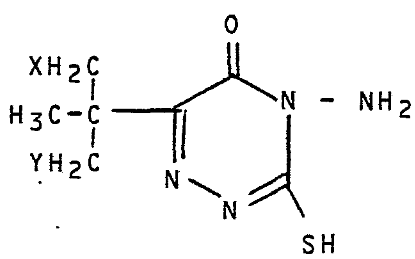

(II)

in welcher

X und Y die oben angegebene Bedeutung haben,

mit Halogeniden der Formel III

$$Hal - R^1 \qquad (III)$$

in welcher

$R^1$ die oben angegebene Bedeutung hat und

Hal für Halogen steht,

in Gegenwart einer Base und gegebenenfalls in Gegenwart eines Verdünnungsmittels umsetzt, oder

b) 3-Alkylmercapto-4-amino-6-tert.-butyl-1,2,4-triazin-5-one der Formel IV

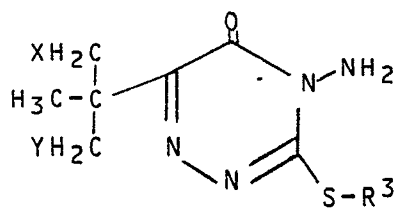

(IV)

in welcher

X und Y die oben angegebene Bedeutung haben und

$R^3$ für Alkyl steht,

mit Aminen der Formel V

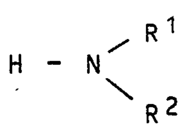

(V)

in welcher

$R^1$ und $R^2$ die oben angegebene Bedeutung haben,

in Gegenwart eines Verdünnungsmittels und gegebenenfalls in Gegenwart einer niederen aliphatischen Carbonsäure umsetzt.

Außerdem wurde gefunden, daß die 3-Alken(in)yl-mercapto-(amino)-4-amino-6-tert.-butyl-1,2,4-triazin-5-one der Formel (I) gute herbizide, insbesondere selektiv-herbizide Eigenschaften aufweisen.

Ueberraschenderweise zeigen die erfindungsgemäßen Verbindungen gegenüber dem bekannten 4-Amino-3-methylmercapto-6-tert.-butyl-1,2,4-triazin-5-on, welches chemisch und wirkungsmäßig eine naheliegende Verbindung ist, bei gleich guter allgemeiner herbizider Wirkung eine deutlich bessere Verträglichkeit bei wichtigen Kulturpflanzen, wie insbesondere bei Weizen, Hafer und Mais. Die erfindungsgemäßen Wirkstoffe stellen somit eine wesentliche Bereicherung der herbiziden Mittel, insbesondere der selektiven chemischen Unkrautbekämpfung dar.

Die erfindungsgemäßen 3-Alken(in)yl-mercapto(amino)-4-amino-6-tert.-butyl-1,2,4-triazin-5-one sind durch die Formel (I) allgemein definiert. Bevorzugt sind diejenigen Verbindungen, in denen

A für Schwefel oder den Rest $-NR^2-$ steht, wobei

$R^2$ für Wasserstoff oder geradkettiges und verzweigtes Alkyl mit 1 bis 4 Kohlenstoffatomen steht;

$R^1$ für geradkettiges oder verzweigtes Alkenyl mit 3 bis 6 Kohlenstoffatomen und geradkettiges oder verzweigtes Alkinyl mit 3 bis 6 Kohlenstoffatomen steht;

X für Fluor, Chlor, Brom und geradkettiges oder verzweigtes Alkoxy mit 1 bis 4 Kohlenstoffatomen steht; und

Y für Wasserstoff, Fluor, Chlor, Brom und geradkettiges oder verzweigtes Alkoxy mit 1 bis 4 Kohlenstoffatomen steht.

Besonders bevorzugt sind diejenigen Verbindungen der Formel (I), in denen

A für Schwefel oder den Rest $-NR^2-$ steht, wobei

$R^2$ für Wasserstoff, Methyl oder Ethyl steht;

$R^1$ für die Reste: $-CH_2-CH=CH_2$, $-CH_2-CH=CH-CH_3$, $-CH_2-C(CH_3)=CH_2$, $-CH(CH_3)-CH=CH_2$, $-C(CH_3)_2-CH=CH_2$, $-CH_2C\equiv CH$, $-CH(CH_3)-C\equiv CH$ und $-C(CH_3)_2-C\equiv CH$ steht;

X für Fluor, Chlor, Methoxy, Ethoxy oder Isopropoxy steht; und

Y für Wasserstoff, Fluor, Chlor, Methoxy, Ethoxy oder Isopropoxy steht.

Verwendet man beispielsweise 4- Amino-6-chlor-tert.-butyl-3-mercapto-1,2,4-triazin-5-on und Propargylbromid als Ausgangsstoffe, so kann der Reaktionsablauf gemäß Verfahren (a) durch das folgende Formelschema wiedergegeben werden:

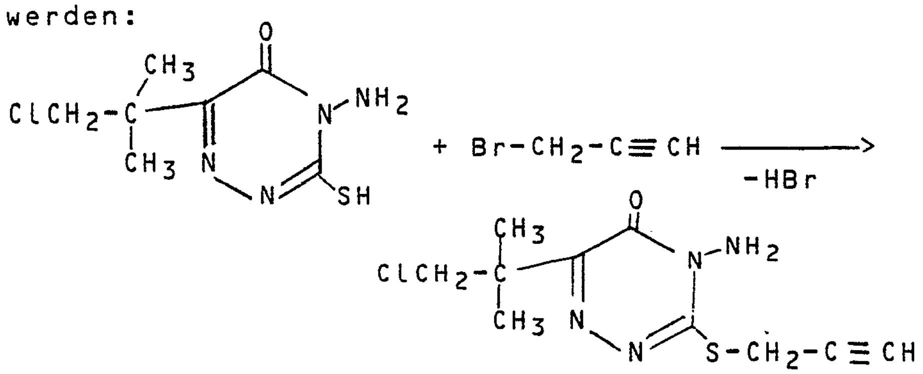

Verwendet man beispielsweise 4-Amino-6-fluor-tert.-butyl-3-methylthio-1,2,4-triazin-5-on und Allylamin als Ausgangsstoffe, so kann der Reaktionsablauf gemäß Verfahren (b) durch das folgende Formelschema wiedergegeben werden:

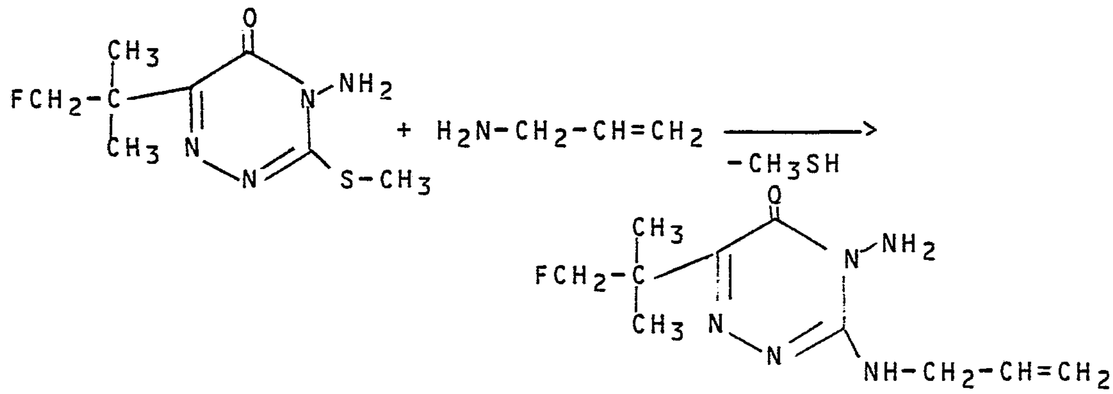


Die für das Verfahren (a) als Ausgangsstoffe zu verwendenden 4-Amino-3-mercapto-6-tert.-butyl-1,2,4-triazin-5-one sind durch die Formel (II) allgemein definiert. In dieser Formel stehen X und Y vorzugsweise für die Reste, die bereits im Zusammenhang mit der Beschreibung der erfindungsgemäßen Stoffe der Formel (I) vorzugsweise für diese Substituenten genannt wurden.

Die 4-Amino-3-mercapto-6-tert.-butyl-1,2,4-triazin-5-one der Formel (II) sind noch nicht bekannt; sie sind jedoch Gegenstand eigener älterer Patentanmeldungen, die noch nicht veröffentlicht sind (vergleiche die Deutschen Patentanmeldungen P 30 37 300 vom 2.10.1980 [LeA 20 631] und P 31 35 392 vom 7.9.1981 [LeA 21 175]). Sie werden erhalten, indem man in einer <u>ersten Stufe</u> Pivaloylcyanide der Formel VI

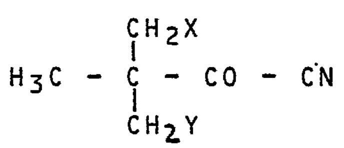

(VI)

in welcher

X und Y die oben angegebene Bedeutung haben,

(α) gegebenenfalls in Gegenwart einer flüssigen Carbonsäure als Lösungsmittel, wie beispielsweise Essigsäure, mit einer anorganischen Säure, wie beispielsweise einer Halogenwasserstoffsäure, bei Temperaturen zwischen 0 und 40°C umsetzt, oder

(ß) an ein Carboniumion addiert, das aus einem Olefin, wie z.B. Isobutylen, oder einem tertiären Alkohol, wie z.B. tert.-Butanol, mit einer starken Säure, wie insbesondere Schwefelsäure, gebildet wird, und anschließend der Hydrolyse unterwirft,

und die hierbei entstehenden Trimethylbrenztraubensäureamide der Formel VII a

$$\begin{array}{c} \quad\;\; CH_2X \\ \quad\;\; | \\ H_3C - C - CO - CO - NHR \\ \quad\;\; | \\ \quad\;\; CH_2Y \end{array} \qquad (VIIa)$$

in welcher

X und Y die oben angegebene Bedeutung haben und

R für Wasserstoff (erhältlich nach Variante α) oder Alkyl, insbesondere tert.-Butyl (erhältlich nach Variante ß), steht,

in einer <u>zweiten Stufe</u> in an sich bekannter Weise entweder direkt in der anfallenden Lösung oder nach Zwischenisolierung, gegebenenfalls nach vorheriger Verseifung zu den freien substituierten Trimethylbrenztraubensäuren der Formel VII b

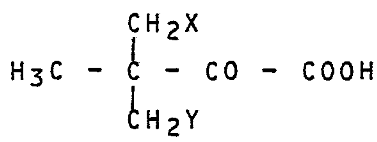

(VII b)

in welcher

X und Y die oben angegebene Bedeutung haben,

mit Thiocarbohydrazid der Formel VIII

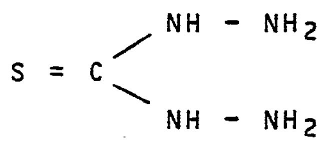

(VIII)

in wässriger bzw. in wässrig-saurer Lösung, wie einer halogenwasserstoffsauren Lösung, gegebenenfalls in Gegenwart eines organischen Lösungsmittels, wie insbesondere Dimethylformamid, bei Temperaturen zwischen 0 und 100°C umsetzt.

Die Pivaloylcyanide der Formel (VI) sind noch nicht bekannt. Auch sie sind Gegenstand der o.g. eigenen älteren Deutschen Patentanmeldungen, die noch nicht veröffentlicht sind. Die Pivaloylcyanide der Formel (VI) können erhalten werden, indem man die entsprechenden Pivaloylhalogenide bzw. -anhydride der Formeln IXa bzw. IXb

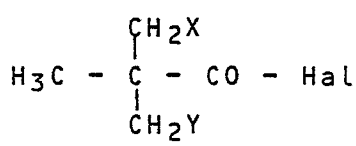

(IXa)

bzw.

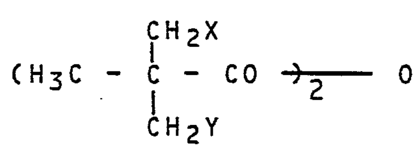

(IX b)

in welchen

X und Y die oben angegebene Bedeutung haben und

Hal für Halogen, vorzugsweise Chlor oder Brom, steht,

mit Trimethylsilylcyanid, gegebenenfalls in Gegenwart eines inerten organischen Lösungsmittels, wie beispielsweise Acetonitril, bei Temperaturen zwischen 80 und 110°C umsetzt. Das Trimethylsilylcyanid, $(CH_3)_3Si-CN$, ist bekannt (vergleiche z.B. Synthesis 1979, Seiten 522 und 523).

Die Pivaloylhalogenide bzw. -anhydride der Formeln (IXa) und (IX b) sind bekannt, bzw. können sie nach bekannten Verfahren hergestellt werden.

Die außerdem für das Verfahren (a) als Ausgangsstoffe zu verwendenden Halogenide sind durch die Formel (III) allgemein definiert. In dieser Formel steht $R^1$ vorzugsweise für die Reste, die bereits im Zusammenhang mit der Beschreibung der erfindungsgemäßen Stoffe der Formel (I) vorzugsweise für diesen Substituenten genannt wurden. Hal steht vorzugsweise für Chlor oder Brom.

Die Halogenide der Formel (III) sind allgemein bekannte Verbindungen der organischen Chemie.

Die für das Verfahren (b) als Ausgangsstoffe zu verwendenden 3-Alkylmercapto-4-amino-6-tert.-butyl-1,2,4-triazin-5-one sind durch die Formel (IV) allgemein definiert. In dieser Formel stehen X und Y vorzugsweise für die Reste, die bereits im Zusammenhang mit der Beschreibung der erfindungsgemäßen Stoffe der Formel (I) vorzugsweise für diese Substituenten genannt wurden. $R^3$ steht vorzugsweise für Alkyl mit 1 bis 4 Kohlenstoffatomen, wie insbesondere für Methyl.

Die 3-Alkylmercapto-4-amino-6-tert.-butyl-1,2,4-triazin-5-one der Formel (IV) sind noch nicht bekannt. Auch sie

sind Gegenstand der beiden oben genannten Deutschen Patentanmeldungen, die noch nicht veröffentlicht sind, und werden erhalten, indem man 4-Amino-3-mercapto-6-tert.- butyl-1,2,4-triazin-5-one der Formel (II) entsprechend der Verfahrensvariante (a) mit Alkylhalogenid, wie beispielsweise Methyliodid oder -bromid, umsetzt.

Die außerdem für das Verfahren (b) als Ausgangsstoffe zu verwendenden Amine sind durch die Formel (V) allgemein definiert. In dieser Formel stehen $R^1$ und $R^2$ vorzugsweise für die Reste, die bereits im Zusammenhang mit der Beschreibung der erfindungsgemäßen Stoffe der Formel (I) vorzugsweise für diese Substituenten genannt wurden.

Die Amine der Formel (V) sind allgemein bekannte Verbindungen der organischen Chemie.

Für die erfindungsgemäße Umsetzung gemäß Verfahren (a) kommen als Verdünnungsmittel protische und aprotische Lösungsmittel infrage. Hierzu gehören beispielsweise Wasser, Alkohole, Carbonsäuren, Aceton, Acetonitril, Dimethylformamid, Dimethylsulfoxid und Toluol.

Die erfindungsgemäße Umsetzung gemäß Verfahren (a) wird in Gegenwart einer Base durchgeführt. Hierzu gehören alle üblicherweise verwendbaren organischen und insbesondere anorganischen Basen, wie beispielsweise Natrium- und Kaliumhydroxid oder Natrium- und Kaliumcarbonat.

Die Reaktionstemperaturen können bei der Durchführung des erfindungsgemäßen Verfahrens (a) in einem größeren Bereich variiert werden. Im allgemeinen arbeitet man zwischen 0 und 120°C, vorzugsweise zwischen 20 und 100°C.

Bei der Durchführung des erfindungsgemäßen Verfahrens setzt man auf 1 Mol der Verbindung der Formel (II) vorzugsweise 1 bis 2 Mol Halogenid der Formel (III) ein. Die Isolierung der Endprodukte erfolgt in allgemein üblicher Art und Weise.

Nach einer bevorzugten Ausführungsform des erfindungsgemäßen Verfahrens (a) wird die Umsetzung in einem Zweiphasensystem, wie beispielsweise wässrige Natron- oder Kalilauge/Toluol oder Methylenchlorid, unter Zusatz von 0,01 - 1 Mol eines Phasen-Transfer-Katalysators, wie beispielsweise Ammonium- oder Phosphoniumverbindungen, pro Mol der Verbindung (II), durchgeführt.

Für die erfindungsgemäße Umsetzung gemäß Verfahren (b) kommen als Verdünnungsmittel alle inerten organischen Lösungsmittel infrage. Hierzu gehören Kohlenwasserstoffe wie Toluol, Xylol; chlorierte aromatische Kohlenwasserstoffe wie Chlorbenzol, 1,2-Dichlorbenzol, 1,2,4-Trichlorbenzol; Ether, wie Tetrahydrofuran, Dioxan; Alkohole wie Methanol, Ethanol, Propanol, Isopropanol; Amide wie N,N-Dimethylformamid, Tetramethylharnstoff oder Sulfoxide wie Dimethylsulfoxid. Vorzugsweise wird für die Umsetzung Isopropanol verwendet.

Die Reaktionstemperaturen können bei der Durchführung des erfindungsgemäßen Verfahrens (b) in einem größeren Bereich variiert werden. Im allgemeinen arbeitet man bei Temperaturen zwischen 60 und 90°C.

Die Reaktion gemäß Verfahren (b) kann drucklos, wie auch bei erhöhten Drucken ausgeführt werden.

Eine besonders vorteilhafte Ausführungsform des Verfahres (b) besteht darin, daß man in Gegenwart mindestens der äquimolaren Menge einer niederen aliphatischen Carbonsäure arbeitet. Vorzugsweise wird hierfür Essigsäure verwendet. Dieses Verfahren gestattet es, mit relativ geringem Amin-Ueberschuß auszukommen. Bei dieser Ausführungsform kann die Reaktionsgeschwindigkeit durch Zusatz einer katalytischen Menge einer organischen Sulfonsäure erhöht werden. Vorzugsweise verwendet man hierfür p-Toluolsulfonsäure.

Bei der Durchführung des erfindungsgemäßen Verfahrens (b) nach dieser bevorzugten Verfahrensvariante setzt man auf 1 Mol des 3-Alkylmercaptotriazinons der Formel (IV) zweckmäßigerweise 1 bis 2 Mol einer niederen aliphatischen Carbonsäure, 0,01 bis 0,05 Mol einer organischen Sulfonsäure und 1 bis 5 Mol Amin der Formel (V) ein, erhitzt bis zum Ende der Mercaptan-Abspaltung und arbeitet anschließend auf.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum,Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Wein-, Citrus-, Nuss-, Bananen-, Kaffee-, Tee-, Gummi-, Oelpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbiziden Wirkung eine gute Verträglichkeit gegenüber Nutzpflanzen. So ist es möglich, wichtige Schadgräser selektiv in wichtigen Kulturpflanzen, wei z.B. in Weizen, Hafer und Mais zu bekämpfen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen oder Stäuben.

Die erfindungsgemäßen Wirkstoffe können bei einer Anwendung als Herbizide sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen ab von der Art des gewünschten Effektes. Im allgemeinen liegen die Aufwandmengen zwischen 0,1 und 10 kg/ha, vorzugsweise zwischen 0,2 und 6 kg/ha. Die nachfolgenden Beispiele dienen zur weiteren Erläuterung der Erfindung.

Herstellungsbeispiele

Beispiel 1

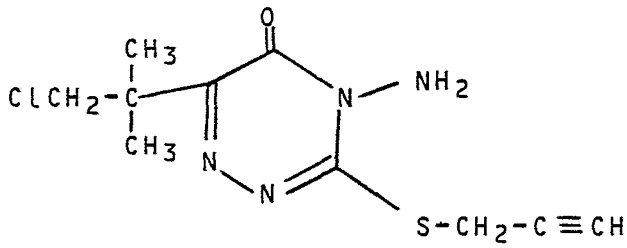

(Verfahren a)

Zu einer Lösung von 23,5g (0,1 Mol) 4-Amino-6-chlor-tert.-butyl-3-mercapto-1,2,4-triazin-5-on und 4,4g Natriumhydroxid in 40 ml Wasser werden 18,6g (80%-ige Lösung in Toluol= 0,125 Mol) Propargylbromid und 0,5 g Pentyltributylphosphoniumbromid gegeben. Man läßt das Reaktionsgemisch ca. 20 Stunden bei Raumtemperatur rühren, versetzt anschließend mit 100 ml Toluol und filtriert. Das Filtrat wird mit 100 ml Wasser, mit 100 ml 1n Natronlauge und nochmals mit Wasser gewaschen. Die organische Phase wird über Natriumsulfat getrocknet, filtriert und eingeengt. Der ölige Rückstand wird durch Verreiben in Diisopropylether zur Kristallisation gebracht und aus Essigester /Petrolether umkristallisiert. Man erhält 13,8g (57,5 % der Theorie) 4-Amino-6-chlor-tert.-butyl-3-propargylthio-1,2,4-triazin-5-on vom Schmelzpunkt 107-108°C.

Herstellung des Ausgangsproduktes

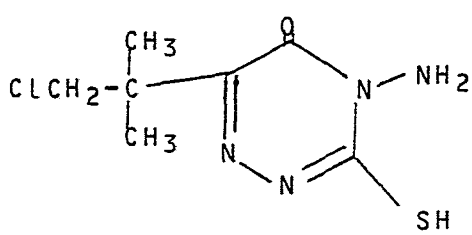

Zu 9 l einer Lösung von Bromwasserstoff in Eisessig (33%-ig) werden unter Rühren 2,31 kg (15,88 Mol) Chlorpivaloylcyanid bei Raumtemperatur gegeben. Man läßt 4 Stunden bei Raumtemperatur nachrühren. Anschließend werden bei 7 bis 10°C 288 ml (15,88 Mol) Wasser zugegeben (exotherme Reaktion, ca.37°C) und 3 Stunden bei Raumtemperatur nachgerührt. Die Reaktionslösung wird danach bei 7 bis 10°C in eine Mischung von 2,03 kg Thiocarbohydrazid und 15,9 l 1n Salzsäure eingetragen (stark exotherme Reaktion). Dieses Reaktionsgemisch wird 2 Stunden bei 7 bis 10°C und 14 Stunden bei Raumtemperatur nachgerührt. Danach wird das ausfallende Kristallisat abfiltriert, mit Wasser gewaschen und getrocknet. Man erhält 2995g (80,4 % der Theorie) rohes 4-Amino-6-chlor-tert.-butyl-3-mercapto-1,2,4-triazin-5-on vom Schmelzpunkt 202-208°C.

$$\begin{array}{c} \qquad\quad CH_3 \\ \qquad\quad | \\ ClCH_2 - C - CO - CN \\ \qquad\quad | \\ \qquad\quad CH_3 \end{array}$$

8300 g (49,5 Mol) 92,5 % -iges ß-Chlorpivaloylchlorid werden auf 100°C erwärmt und innerhalb von etwa 2 Stunden mit 4950g (50 Mol) Trimethylsilylcyanid versetzt. Das entstehende Trimethylsilylchlorid wird gleichzeitig abdestilliert. Nach beendeter Zugabe wird die Temperatur langsam auf 140°C gesteigert und etwa 1,5 Stunden bei dieser Temperatur gerührt. Anschließend wird die Reaktionsmischung im Vakuum destilliert. Man erhält 7500g ß-Chlorpivaloylcyanid vom Siedepunkt 62-65°C/16 mbar.

Beispiel 2

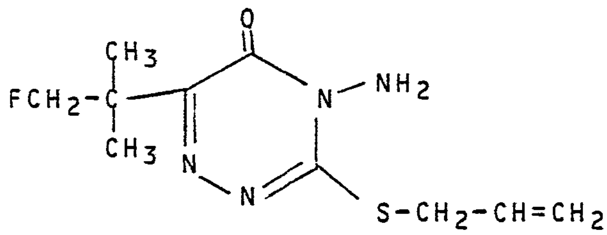

(Verfahren a)

Eine Lösung von 43,6g (0,2 Mol) 4-Amino-6-fluor-tert.-butyl-3-mercapto-1,2,4-triazin-5-on in 200 ml 1n Natronlauge wird bei Raumtemperatur mit 26,6g (0,22 Mol) Allylbromid versetzt. Man läßt das Reaktionsgemisch ca. 20 Stunden bei Raumtemperatur rühren, nimmt anschließend die organische Phase in Chloroform auf, wäscht dreimal mit je 150 ml 0,1 n Natronlauge, trocknet über Natriumsulfat, filtriert und engt ein. Der ölige Rückstand wird bei 50°C/0,05 mbar entgast und aus Essigester/Petrolether umkristallisiert. Man erhält 20,8 g (40 % der Theorie) 3-Allylthio-4-amino-6-fluor-tert.-butyl-1,2,4-triazin-5-on vom Schmelzpunkt 73-74°C.

Beispiel 3

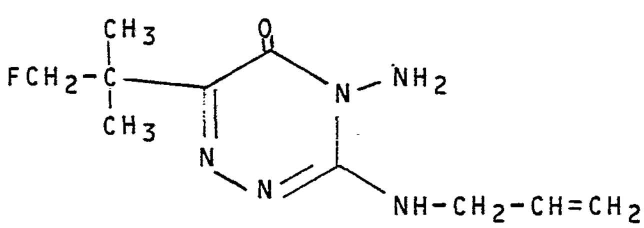

(Verfahren b)

11,6g (0,05 Mol) 4-Amino-6-fluor-tert.-butyl-3-methylthio-1,2,4-triazin-5-on und 14,3g (0,25 Mol) Allylamin werden zu 6g (0,1 Mol) Eisessig in 250 ml Isopropanol gegeben und unter Eiskühlung verrührt. Danach wird das Reaktionsgemisch ca. 40 Stunden unter Rückfluß erhitzt. Man

läßt abkühlen, engt ein, nimmt den Rückstand in Methylenchlorid auf, wäscht mit 200 ml 1n Natronlauge und mit 200 ml Wasser, trocknet über Natriumsulfat, filtriert und engt ein. Der ölige Rückstand kristallisiert durch Verreiben mit Diisopropylether. Nach Umkristallisation aus Essigester/Petrolether erhält man 4,9 g ( 41 % der Theorie) 3-Allylamino-4-amino-6-fluor-tert.-butyl-1,2,4-triazin-5-on vom Schmelzpunkt 100-104°C.

Herstellung des Ausgangsproduktes

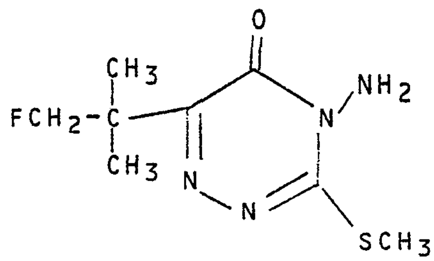

Entsprechend Beispiel 1 erhält man, ausgehend von Fluorpivaloylcyanid, in 80%iger Ausbeute zunächst rohes 4-Amino-6-fluor-tert.-butyl-3-mercapto-1,2,4-triazin-5-on. 3347,4 g (12 Mol) des so erhaltenen 4-Amino-6-fluor-tert.-butyl-3-mercapto-1,2,4-triazin-5-ons werden in 15 l 1n Natronlauge gelöst. Nach vollständiger Lösung des Produktes werden bei 7 bis 10°C 1873,6g Methyliodid zugetropft. Nach beendeter Zugabe läßt man 2 Stunden bei 7 bis 10°C und über Nacht bei Raumtemperatur nachrühren. Danach wird der entstandene Feststoff abgesaugt, mit Wasser gewaschen und getrocknet. Man erhält 2285g (65% der Theorie) 4-Amino-6-fluor-tert.-butyl-3-methylthio-1,2,4-triazin-5-on vom Schmelzpunkt 121-122°C.

In analoger Weise und entsprechend den erfindungsgemäßen Verfahren werden die in der nachfolgenden Tabelle 1 aufgeführten Verbindungen der allgemeinen Formel

Tabelle 1

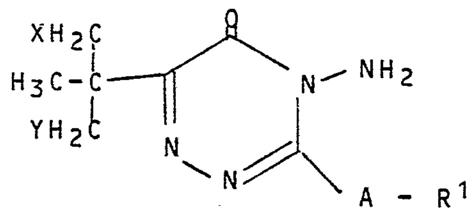

(I)

erhalten:

| Bsp. Nr. | X | Y | A | $R^1$ | Schmelzpunkt (°C) oder $n_D^{20}$ |
|---|---|---|---|---|---|
| 4 | F | H | S | $-CH_2-CH=CH-CH_3$ | 1,566 |
| 5 | F | H | S | $-CH_2-C\equiv CH$ | 94-95 |
| 6 | Cl | H | S | $-CH_2-CH=CH_2$ | 65-66 |
| 7 | Cl | H | S | $-CH_2-CH=CH-CH_3$ | 1,577 |
| 8 | Cl | H | NH | $-CH_2-CH=CH_2$ | 82-85 |
| 9 | F | F | S | $-CH_2-CH=CH-CH_3$ | 1,555 |
| 10 | Cl | H | NH | $-CH_2-C\equiv CH$ | 116-17 |
| 11 | F | H | NH | $-CH_2-C\equiv CH$ | 129-31 |
| 12 | F | H | $N(CH_3)$ | $-CH_2-C\equiv CH$ | zähes Öl |
| 13 | F | H | S | $-CH_2-CH=CH_2$ | 1,550 |
| 14 | F | H | S | $-CH_2-C\equiv CH$ | 68-69 |
| 15 | Cl | H | $N(CH_3)$ | $-CH_2-C\equiv CH$ | 1,562 |
| 16 | F | F | NH | $-CH_2-CH=CH_2$ | 101-102 |
| 17 | $H_5C_2O-$ | H | S | $-CH_2-C\equiv CH$ | 69-70 |
| 18 | $H_3CO-$ | H | S | $-CH_2-C\equiv CH$ | 133-34 |
| 19 | $H_5C_2O-$ | H | S | $-CH_2-CH=CH_2$ | 54-56 |
| 20 | $H_5C_2O-$ | H | NH | $-CH_2-CH=CH_2$ | 1,536 |
| 21 | $H_5C_2O-$ | H | S | $-CH_2-CH=CH-CH_3$ | 1,557 |

Verwendungsbeispiele:

Beispiel A

Pre-emergence-Test

Lösungsmittel: 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in derZubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen z.B. die Verbindungen gemäß der Herstellungsbeispiele 1,3,5 und 8 bei guter allgemeiner Wirksamkeit eine sehr gute Selektivität in Weizen und Mais.

Beispiel B

Post-emergence-Test

Lösungsmittel: 5 Gewichtsteile Aceton

Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubetreitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen z.B. die Verbindungen gemäß der Herstellungsbeispiele 1,3 und 5 bei guter allgemeiner Wirksamkeit eine sehr gute Selektivität in Hafer, Weizen und Mais.

Patentansprüche

1) 3-Alken(in)yl-mercapto(amino)-4-amino-6-tert.-butyl-1,2,4-triazin-5-one der allgemeinen Formel I

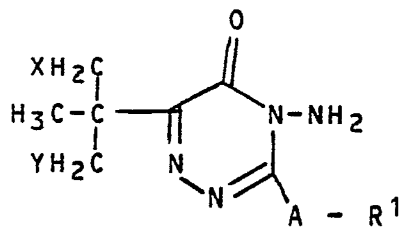

(I)

in welcher

A für Schwefel oder den Rest $-NR^2-$ steht,

$R^2$ für Wasserstoff oder Alkyl steht,

$R^1$ für Alkenyl oder Alkinyl steht,

X für Halogen oder Alkoxy steht und

Y für Wasserstoff, Halogen oder Alkoxy steht.

2) Verfahren zur Herstellung von 3-Alken(in)yl-mercapto(amino)-4-amino-6-tert.-butyl-1,2,4-triazin-5-onen der allgemeinen Formel(I) gemäß Anspruch 1, dadurch gekennzeichnet, daß man

a) 4-Amino-3-mercapto-6-tert.-butyl-1,2,4-triazin-5-one der Formel II

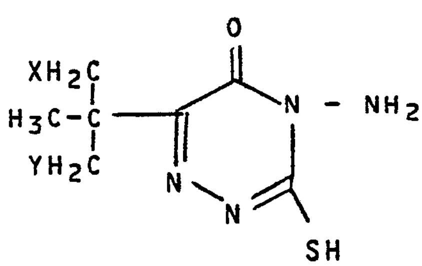

(II)

in welcher

X und Y die oben angegebene Bedeutung haben.

mit Halogeniden der Formel III

Hal - $R^1$ (III)

in welcher

$R^1$ die oben angegebene Bedeutung hat und

Hal für Halogen steht,

in Gegenwart einer Base und gegebenenfalls in Gegenwart eines Verdünnungsmittels umsetzt, oder

b) 3-Alkylmercapto-4-amino-6-tert.-butyl-1,2,4-triazin-5-one der Formel IV

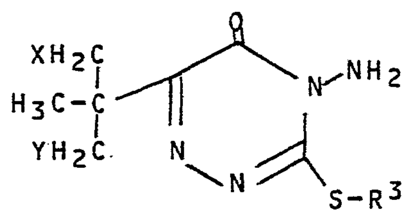

(IV)

in welcher

X und Y die oben angegebene Bedeutung haben und

$R^3$ für Alkyl steht,

mit Aminen der Formel V

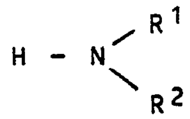

(V)

in welcher

$R^1$ und $R^2$ die oben angegebene Bedeutung haben,

in Gegenwart eines Verdünnungsmittels und gegebenenfalls in Gegenwart einer niedrigen aliphatischen Carbonsäure umsetzt.

3) Herbizide Mittel, gekennzeichnet durch einen Gehalt an mindestens einem 3-Alken(in)yl-mercapto(amino)-4-amino-6-tert.-butyl-1,2,4-triazin-5-on der Formel(I) gemäß Anspruch 1.

4) Verwendung von 3-Alken(in)yl-mercapto(amino)-4-amino-6-tert.-butyl-1,2,4-triazin-5-onen der Formel (I) gemäß Anspruch 1 zur Bekämpfung von unerwünschtem Pflanzenwachstum.

5) Verfahren zur Herstellung von herbiziden Mitteln, dadurch gekennzeichnet, daß man 3-Alken(in)yl-mercapto-(amino)-4-amino-6-tert.-butyl-1,2,4-triazin-5-one der Formel (I) gemäß Anspruch 1 mit Streckmitteln und/oder oberflächenaktiven Mitteln vermischt.

6) 3-Alken(in)-yl-mercapto(amino)-4-amino-6-tert.-butyl-1,2,4-triazin-5-one der Formel (I) gemäß Anspruch 1, dadurch gekennzeichnet daß

A für Schwefel oder den Rest $-NR^2-$ steht, wobei

$R^2$ für Wasserstoff oder geradkettiges und verzweigtes Alkyl mit 1 bis 4 Kohlenstoffatomen steht;

$R^1$ für geradkettiges oder verzweigtes Alkenyl mit 3 bis 6 Kohlenstoffatomen und geradkettiges oder verzweigtes Alkinyl mit 3 bis 6 Kohlenstoffatomen steht;

X für Fluor, Chlor, Brom und geradkettiges oder verzweigtes Alkoxy mit 1 bis 4 Kohlenstoffatomen steht;

Y für Wasserstoff, Fluor, Chlor, Brom und geradkettiges oder verzweigtes Alkoxy mit 1 bis 4 Kohlenstoffatomen steht.

7) 4-Amino-6-(chlor-tert.-butyl)-3-propargylthio-1,2,4-triazin-5-on (1) gemäß Anspruch 1.

8) 3-Allylamino-4-amino-6-(fluor-tert.-butyl)-1,2,4-triazin-5-on (3) gemäß Anspruch 1

9) 4-Amino-6-(fluor-tert.-butyl)-3-propargylthio-1,2,4-triazin-5-on (5) gemäß Anspruch 1.

10) 3-Allylamino-4-amino-6-(chlor-tert.-butyl)-1,2,4-triazin-5-on (8) gemäß Anspruch 1.

0084774

Europäisches Patentamt

**EUROPÄISCHER RECHERCHENBERICHT**

Nummer der Anmeldung

EP 83 10 0044

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | US-A-3 671 523 (BAYER) * Spalte 1, Zeilen 30-31, 41-45, 50-51; Spalte 2, Zeile 5-34; Spalte 20, Nr. 150,156,158; Spalte 21, Nr. 179; Patentansprüche * --- | 1-6 | C 07 D 253/06<br>A 01 N 43/64 //<br>C 07 C 121/34 |
| P,A | EP-A-0 049 416 (BAYER) * Insgesamt * ----- | 1-6 | |
| | | | **RECHERCHIERTE SACHGEBIETE (Int. Cl. 3)** |
| | | | C 07 D 253/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 13-04-1983 | NUYTS A.M.K.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82